# FASCICULE DE BREVET EUROPEEN

(11) **EP 1 128 829 B1**
(45) Date de publication et mention de la délivrance du brevet: **11.05.2005**
(21) Numéro de dépôt: 99972101.2
(22) Date de dépôt: 09.11.1999
(51) Int. Cl.: A61K 31/425, A61K 31/355

(54) **ASSOCIATION DE RILUZOLE ET D'ALPHA-TOCOPHEROL**
ZUSAMMENSETZUNG VON RILUZOL UND ALPHA-TOCOPHEROL
RILUZOLE AND ALPHA-TOCOPHEROL COMBINATION

(30) Priorité: 13.11.1998 FR 9814250
(43) Date de publication de la demande: 05.09.2001
(73) Titulaire: Aventis Pharma S.A., 92160 Antony (FR)
(72) Inventeur: DIB, Michel, F-75013 Paris (FR)
(74) Mandataire: Rousseau, Pierrick Edouard
(86) Numéro de dépôt international: PCT/FR1999/002753
(87) Numéro de publication internationale: WO 2000/028992

(56) Documents cités:
- ROSS M.A.: "Acquired motor neuron disorders" NEUROLOGIC CLINICS (NEUROL. CLIN.), 1997, 15/3 (481-500), XP002108929 United States
- GURNEY ME ET AL: "Pathogenic mechanisms in familial amyotrophic lateral sclerosis due to mutation of Cu, Zn superoxide dismutase." PATHOL BIOL (PARIS), JAN 1996, 44 (1) P51-6, XP002108930 FRANCE
- MILLER R.G.: "New approaches to therapy of amyotrophic lateral sclerosis" WESTERN JOURNAL OF MEDICINE (WEST. J. MED.), 168/4 (262-263), XP002108931 United States
- FLECK T.J. ET AL: 'CHRONIC TREATMENT STUDIES IN TRANSGENIC ALS MICE WITH THE GLUTAMATERELEASE INHIBITOR RILUZOLE AND THE ANTIOXIDANTS VITAMIN E AND SELENIUM' SOCIETY FOR NEUROSCIENCE ABSTRACTS, SOCIETY FOR NEUROSCIENCE, US vol. 23, no. 1,
- GURNEY M.E. ET AL: 'BENEFIT OF VITAMIN E, RILUZOLE, AND GABAPENTIN IN A TRANSGENIC MODEL OF FAMILIAL AMYOTROPHIC LATERAL SCLEROSIS' TRANSACTIONS OF THE AMERICAN NEUROLOGICAL ASSOCIATION, SPRINGER, NEW YORK vol. 39, no. 2, pages 147 - 157

## Description

La présente invention concerne l'association d'α-tocophérol et de riluzole ou d'un sel pharmaceutiquement acceptable de ce composé et l'utilisation de cette association pour le traitement de la sclérose latérale amyotrophique (SLA).

La sclérose latérale amyotrophique également connue sous le nom de maladie de CHARCOT et maladie de LOU GEHRIG a été décrite pour la première fois par CHARCOT en 1865. La SLA est une maladie mortelle résultant de la dégénérescence des motoneurones. La maladie s'accompagne d'une paralysie progressive, conduisant à la perte des fonctions motrices et respiratoires puis à la mort dans un délai de deux à huit ans après l'apparition des premiers symptômes.

Le document Acquired motor neuron disorders de la revue Neurologic Clinics 1997, **15**, 3, 481-500 de M. A. Ross fait un bilan sur la SLA et décrit particulièrement un essai clinique avec des patients qui reçoivent des hautes doses de vitamine E et C et qui après traitement ne présentent pas d'amélioration. Le bénéfice bien que reconnu pour la souris transgénique n'a pas été retrouvé chez l'homme.
Le document Pathogenic mechanism in familial amyotrophic lateral sclerosis due to mutation of Cu, Zn superoxide dismutase dans Pathologie Biologie, 1996, **44**, 51-56 de M. E. Gurney démontre que l'apport de vitamine E chez la souris transgenique retarde l'apparition des symptomes de la SLA mais ne prolonge pas la survie. Le document New Approach to therapy of Amyotrophic Lateral Sclerosis dans Epitome Neurology 1998, **168**, 262-263 de R. G. Miller décrit que les souris transgénique ayant reçu de la vitamine E retarde l'apparition de ALS.

A ce jour, seul le riluzole (2-amino-6-trifluorométhoxybenzothiazole) est commercialisé sous le nom de RILUTEK^{R} pour le traitement de la sclérose latérale amyotrophique. Le riluzole permet principalement de ralentir la progression de la maladie.

Il a maintenant été trouvé que l'association de riluzole ou un de ses sels pharmaceutiquement acceptables et d'α-tocophérol (vitamine E) permet de ralentir la maladie d'une manière plus importante que le riluzole seul et aussi de diminuer la fatigue des patients et la concentration plasmatique de malondialdéhyde.

L'étude a été réalisée chez 289 patients au total âgés de plus de 18 ans, atteints de SLA depuis moins de 5 ans et dont le rapport de la capacité vitale/ la capacité vitale normale théorique est supérieur ou égal à 60% (la capacité vitale est une mesure classique de routine de la fonction respiratoire également appelée épreuve fonctionnelle respiratoire).

Les patients sont divisés en 2 groupes :
groupe 1 : 145 patients traités avec 100 mg/jour de riluzole par voie orale et 1000 mg/jour d'α-tocophérol par voie orale,
groupe 2 : 144 patients traités avec 100 mg/jour de riluzole par voie orale et du placébo.

Certains patients des 2 groupes ne répondant plus, dans une deuxième évaluation, aux critères d'inclusion ou bien n'ayant pas suivi le traitement correctement, n'ont pas été pris en compte pour la détermination des résultats.

Les malades sont suivis pendant une année. Les résultats sont mesurés sur des échelles fonctionnelles (stades fonctionnels de Munsat (RIVIERE et coll., Arch. Neurol., 55, 526 (1998)), échelle visuelle analogique (EVA) pour la fatigabilité (LACOMBLEZ et coll., The Lancet, 347, 1425 (1996); BENSIMON et coll., New England Journal of Medicine, 330, 585 (1994)) et concentration plasmatique du malondialdéhyde, un marqueur biochimique du stress oxydatif (FAVIER, Analysis of free radicals in biological systems, Birk Hauser, Basel/Switzerland, 1995 p 100-117)).

Le stade fonctionnel A de Munsat concerne les patients de stade moyen ou modéré :
- stade moyen : déficit moyen dans 3 régions (parole, bras et jambe), fonctionnellement indépendant dans la parole, activités des extrémités supérieures dans la vie quotidienne et ambulation,
- stade modéré : déficit moyen dans 3 régions ou déficit modéré à sévère dans une région alors que les 2 autres régions sont normales ou légérement affectées.

Le stade fonctionnel B de Munsat concerne les patients de stade sévère ou final :
- stade sévère : nécessité d'assistance dans 2 ou 3 régions, parole dysarthrique et/ou patients nécessitant de l'assistance pour marcher et/ou nécessitant de l'assistance pour les activités des extrémités supérieures dans la vie quotidienne,
- stade final : utilisation non fonctionnelle d'au moins 2 régions et utilisation modérée ou non fonctionnelle de la troisième région.

L'évolution des stades fonctionnels A et B est déterminée au moment de l'inclusion (M0) et après 12 mois de traitement (M12). Les résultats obtenus sont les suivants :

| | RILUZOLE ET PLACEBO (nombre de patients) | RILUZOLE ET α-TOCOPHEROL (nombre de patients) |
|---|---|---|
| Etat à l'inclusion (M0) | | |
| stade A | 109 | 112 |
| stade B | 10 | 10 |
| Etat après traitement (M12) | | |
| stade A | 56 | 73 |
| stade B | 63 | 49 |

Ces résultats démontrent qu'après 12 mois de traitement avec riluzole et placébo 53 patients (44,5%) présentent une aggravation de la maladie et 66 patients (55,3%) ne présentent pas de changement alors que chez les patients traités avec l'association riluzole et α-tocophérol seulement 39 patients (32%) présentent une aggravation et 83 patients (68%) ne présentent pas de changement.

La progression de la maladie est donc plus diminuée par l'association riluzole et α-tocophérol que par le riluzole seul.

La fatigabilité est mesurée selon l'échelle visuelle analogique (EVA) à l'inclusion des malades (M0) et à 3 mois (M3).

Dans ce test, les valeurs moyennes obtenues sont les suivantes :

| | EVA à M0 (mm) | EVA à M3 (mm) | Δ M0-M3 (mm) |
|---|---|---|---|
| RILUZOLE ET PLACEBO (115 patients) | 48,4 | 65,7 | 17,3 |
| ASSOCIATION RILUZOLE ET α-TOCOPHEROL (118 patients ) | 46,4 | 58,6 | 12,2 |

Ces valeurs démontrent clairement que les patients traités par l'association riluzole et α-tocophérol sont moins fatigués que les patients traités par riluzole et placébo.

La concentration plasmatique du malondialdéhyde qui serait un facteur prédictif de l'évolution de la SLA, a été déterminée au moment de l'inclusion des malades (M0) et à 3 mois (M3).

Les moyennes des concentrations obtenues sont les suivantes :

| | Concentration à M0 (µM) | Concentration à M3 (µM) | Δ M3-M0 (µM) |
|---|---|---|---|
| RILUZOLE ET PLACEBO (65 patients) | 2,94±0,40 | 2,72±0,40 | 0,22±0,40 |
| ASSOCIATION RILUZOLE ET α-TOCOPHEROL (58 patients) | 2,86±0,40 | 2,36±0,30 | 0,50±0,50 |

Ces résultats démontrent que la concentration plasmatique de malondialdéhyde est augmentée chez les patients atteints de sclérose latérale amyotrophique (concentrations à M0) et diminue pendant le traitement mais de manière plus significative avec l'association riluzole et α-tocophérol qu'avec le riluzole seul.

Comme sels pharmaceutiquement acceptables du riluzole peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

L'association peut être employée par voie orale, parentérale ou rectale soit simultanément soit séparément soit de manière étalée dans le temps.

La présente invention concerne également les compositions pharmaceutiques comprenant l'association riluzole et α-tocophérol à l'état pur ou sous forme d'une association avec un ou plusieurs diluants et/ou adjuvants compatibles et pharmaceutiquement acceptables et/ou éventuellement en association avec un autre produit pharmaceutiquement compatible et physiologiquement actif.

Comme compositions solides pour administration orale, peuvent être utilisés des comprimés, des pilules, des poudres (capsules de gélatine, cachets) ou des granulés. Dans ces compositions, les principes actifs sont mélangés à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

Les doses dépendent de l'effet recherché, de la durée du traitement et de la voie d'administration utilisée; elles sont généralement de 10 à 400 mg par jour par voie orale pour un adulte avec des doses unitaires allant de 10 à 200 mg de riluzole et de 250 à 4000 mg par jour par voie orale pour un adulte avec des doses unitaires de 100 à 1000 mg d'α-tocophérol.

D'une façon générale, le médecin déterminera la posologie appropriée en fonction de l'âge, du poids et de tous les autres facteurs propres au sujet à traiter.

## Revendications

1. Association d'α-tocophérol et du riluzole ou d'un sel pharmaceutiquement acceptable de ce dernier.

2. Association selon la revendication 1 dans lesquelles on utilise 10 à 400 parties en poids de riluzole pour 250 à 4000 parties en poids d'α-tocophérol.

3. Association selon l'une des revendications 1 ou 2 comme préparation combinée pour une utilisation simultanée, séparée ou étalée dans le temps.

4. Association selon l'une des revendications 1 à 3 pour le traitement de la sclérose latérale amyotrophique.

5. Composition pharmaceutique constituée d'une association selon la revendication 1 à l'état pur ou en présence de tout diluant ou adjuvant compatible et pharmaceutiquement acceptable.

## Patentansprüche

1. Kombination von α-Tocopherol und Riluzol oder eines pharmazeutisch annehmbaren Salzes hiervon.

2. Kombination nach Anspruch 1, worin 10 bis 400 Gewichtsteile Riluzol für 250 bis 4000 Gewichtsteile α-Tocopherol verwendet werden.

3. Kombination nach den Ansprüchen 1 oder 2 als Kombinationspräparat zur zeitlich gleichzeitigen unabhängigen oder aufeinander folgenden Verwendung.

4. Kombination nach den Ansprüchen 1 bis 3 zur Behandlung der amyotrophischen Lateralsklerose.

5. Pharmazeutische Zusammensetzung, bestehend aus einer Kombination gemäß Anspruch 1 in reinem Zustand oder in Anwesenheit jedes Verdünnungsmittels oder Zusatzmittels, das kompatibel und pharmazeutisch annehmbar ist.

## Claims

1. Combination of α-tocopherol and of riluzole or of a pharmaceutically acceptable salt thereof.

2. Combination according to Claim 1, in which 10 to 400 parts by weight of riluzole are used per 250 to 4000 parts by weight of α-tocopherol.

3. Combination according to either of Claims 1 and 2, as a combined preparation for use simultaneously, separately or spaced out over time.

4. Combination according to one of Claims 1 to 3 for the treatment of amyotrophic lateral sclerosis.

5. Pharmaceutical composition consisting of a combination according to Claim 1 in the pure state or in the presence of any compatible and pharmaceutically acceptable diluent or adjuvant.
